(19) Europäisches Patentamt
European Patent Office
Office européen des brevets

(11) Veröffentlichungsnummer: **0 341 311 A1**

(12) # EUROPÄISCHE PATENTANMELDUNG

Veröffentlicht nach Art. 158 Abs. 3 EPÜ

(21) Anmeldenummer: **88906225.3**

(22) Anmeldetag: **25.05.88**

Daten der zugrundeliegenden internationalen Anmeldung:

(86) Internationale Anmeldenummer:
**PCT/SU88/00115**

(87) Internationale Veröffentlichungsnummer:
**WO88/10268 (29.12.88 88/28)**

(51) Int. Cl.[3]: **C 07 K 7/06**
A 61 K 9/48, A 61 K 35/78
A 61 K 37/02

(30) Priorität: **19.06.87 SU 4262648**

(43) Veröffentlichungstag der Anmeldung:
**15.11.89 Patentblatt 89/46**

(84) Benannte Vertragsstaaten:
**AT BE CH DE FR GB IT LI NL SE**

(71) Anmelder: **VSESOJUZNY KARDIOLOGICHESKY NAUCHNY TSENTR AKADEMII MEDITSINSKIKH NAUK SSSR**
**3 Cherepkovskaya ul, 15a,**
**Moscow, 121552(SU)**

(72) Erfinder: **VINOGRADOV, Valentin Antonovich**
**Khoroshevskoe shosse, 34-38**
**Moscow, 123007(SU)**

(72) Erfinder: **TITOV, Mikhail Ivanovich**
**ul. Osennaya, 2-117**
**Moscow, 121609(SU)**

(72) Erfinder: **POLONSKY, Vladimir Markovich**
**ul. Krasina, 14-25**
**Moscow, 123056(SU)**

(72) Erfinder: **KOROBOV, Nikolai Vasilievich**
**ul. Akademika Bochvara, 6-37**
**Moscow, 123182(SU)**

(72) Erfinder: **SOKOLOV, Anatoly Semenovich**
**Jubileiny pr. 68-55 Moskovskaya obl.**
**Khimki, 141400(SU)**

(72) Erfinder: **YAKUSHEVA, Maria Leonidovna**
**ul. Vinnitskaya, 3-13**
**Moscow, 117192(SU)**

(72) Erfinder: **BESPALOVA, Zhanna Dmitrievna**
**ul. Kuntsevskaya, 1/5-229**
**Moscow, 121351(SU)**

(72) Erfinder: **OVCHINNIKOV, Mikhail Vladimirovich**
**ul. Ostrovityanova, 16-3-26**
**Moscow, 113219(SU)**

(72) Erfinder: **PEKELIS, Boris Leonidovich**
**per. Sivtsev-Vrazhek, 21-36**
**Moscow, 121002(SU)**

(74) Vertreter: **von Füner, Alexander, Dr.**
**Patentanwälte v. Füner, Ebbinghaus, Finck Mariahilfplatz 2&3**
**D-8000 München 90(DE)**

(54) **HEXAPEPTIDE UND DARAUF BASIERENDE PHARMAZEUTISCHE ZUBEREITUNGEN ZUR BEHANDLUNG EROSIVER ODER GESCHWÜRIGER LÄSIONEN DES GASTROINTESTINALTRAKTES.**

(57) Neues Hexapeptid hat folgende Struktur:

Tyr – D – Ala – Gly – Phe – Leu – Glu

Das erfindungsgemässe Arzneimittel für Behandlung von erosion-ulzerösen Schädigungen des Verdauungstraktes setzt sich aus einem Wirkstoff, dem genannten Hexapeptid, und einer pharmazeutischen Trägersubstanz zusammen.

EP 0 341 311 A1

Croydon Printing Company Ltd.

0341311

# HEXAPEPTID UND ARZNEIMITTEL ZUR BEHANDLUNG VON EROSION-ULZERÖSEN SCHÄDIGUNGEN DES VERDAUUNGSTRAKTES AUF SEINER GRUNDLAGE

## Gebiet der Technik

Die vorliegende Erfindung bezieht sich auf das Gebiet der organischen Chemie und zwar betrifft sie ein neues Hexapeptid und ein Arzneimittel für die Behandlung von Erosian-ulzerösen Schädigungen des Verdauungstraktes auf seiner Grundlage.

## Vorhergehender Stand der Technik

Gegenwärtig werden für die Behandlung der Verschlimmerungen der Magangeschwüre und der Zwölffingerdarmgeschwüre Blocker von $H_2$-Rezeptoren des Histamins peroral besonders umfassend angewendet, beispielsweise Zymetidin (Somerville K.M.Langman, Mj.S., "Drugs", 1983, 25, S. 315-330). Das genannte Arzneimittel wird in Form von Tabletten innerhalb von mehreren Monaten verabreicht, seine schnelle Absetzung führt in der Regel zur Wiederkehrung des Magengeschwürs. Die Behandlung mit Zimetidin kann zur Entwicklung einer Reihe von Nebenreaktionen führen: Diarrhoe, Myalgien, allergische Reaktionen, Depression, Gynäkomastie, die durch die Überproduktion von Prolatin hervorgerufen wird, zur Supression der Produzierung des Intrinsic-Faktors und der Resorptionsfähigkeit des Vitamins $B_{12}$, zur Verschlechterung der Sekretionsfunktion der Leber, weshalb die Verwendung von Zimetidin bei Patienten mit Leber- und Nierendysfunktion und bei einigen anderen Kategorien von Patienten unerwünscht ist.

Die Behandlung von Colitis ulcerosa erfolgt gegenwärtig hauptsächlich durch perorale Verabreichung von Sulfasalazin (Gemisch von 5-Aminosalyzilat und Sulfapyridin). Das Arneimittel wird von den Patienten oft schlecht vertragen, bei seiner Verabreichung ist

das Auftreten von Brechreiz, Erbrechen, Kopfschmerzen, allergischen Reaktionen, Leukopenie möglich ("Inflammatory Bowel Diseases, ed. by R.N.Allan, Edinburg; 1983).

Bekannt ist ein Hexapeptid folgender Formel: Tyr-D-Ala-Gly-Phe-Leu-Arg, das seiner Struktur nach der erfindungsgemässen Verbindung (US, A, 4565805) nahe steht und für die Behandlung des Zwölffingerdarmgeschwürs verwendet wird. Das genannte Hexapeptid weist eine stark ausgeprägte antiulzeröse Wirkung nur bei der parenteralen Verabreichung auf und ist bei der peroralen Verabreichung wenig effektiv.

Offenbarung der Erfindung

Das beanspruchte Hexapeptid und das Arzneimittel auf seiner Grundlage sind neu und in der Fachliteratur nicht beschrieben.

Der erfindung liegt die Aufgabe zugrunde, ein neues Hexapeptid und ein Arzneimittel auf seiner Grundlage zu entwickeln, das eine hohe antiulzeröse Wirkung bei seiner peroralen Verabreichung besitzt und keine Nebenreaktionen hat.

Die Aufgabe wurde dadurch gelöst, dass erfindungsgemäss ein neues Hexapeptid angemeldet wird, das folgende Struktur aufweist:

Tyr - D - Ala - Gly - Phe - Leu - Glu.

Das erfindungsgemässe Hexapeptid stellt ein Pulver von weisser Farbe dar, es ist im destillierten Wasser, in physiologischer Lösung, Alkohol gut löslich, in Äther, Äthylazetat, Benzol, Hexan unlöslich ;

$[\alpha]_D^{20} = +29{,}8^\circ$ (c 1,10% Ac OH), $R_f$ : 0,74 (Chloroform : Methanol : 32%ige Essigsäure 60:45:20); 0,42 (Äthylazetat : Pyridin : Essigsäure : Wasser 45:20:6:11), 0,48 (H -Butanol : Essigsäure : Wasser 3:1:1), Angaben der Aminosäure-Analyse : Alanin 1,02 (1), Glyzin I,00 (1), Glutaminsäure 1,04 (1), Leuzin

1,02 (1), Tyrosin 0,99 (1), Phenylalanin 1,03 (1), Angaben der hocheffektiven Flüssig-Chromatografie: Peptid wurde mit einem Berg bei 38,24% des Gradienten in der 12., 16. Minute eluiert, Säule 250 x 4,6 mm, Ultrasphera ODS 5 m, bewegliche Phase A : 0,05 M $KH_2PO_4$, B:$CH_3CN$ grad20 ⟶ 50% B in 20 Minuten, Druck 1500 psi, 1 $ml^3$/min, Detektion bei 214 nm.

Das erfindungsgemässe Hexapeptid besitzt antiulzeröse Wirkung. Das Arzneimittel, das einen Wirkstoff und eine pharmazeutische Trägersubstanz enthält, weist erfindungsgemäss als Wirkstoff das beanspruchte Hexapeptid auf.

Das erfindungsgemässe Arzneimittel kann in einer beliebigen Arzneiform, die für perorale Verabreichung geeignet ist, beispielsweise, in Form von Pulvern oder Tabletten für die enterale Verabreichung in Milch, in Pflanzenfetten beziehungsweise in Lipiden in disperser Form sowie in Form von Kapseln, die einen beliebigen pharmazeutisch geeigneten Fettträger enthalten, verwendet werden. Das erfindungsgemässe Präparat kann ausserdem in Form eines intranasalen Aerosols beziehungsweise in Form von rektalen Zäpfchen verwendet werden.

Vorzugsweise enthält das erfindungsgemässe Präparat einen Wirkstoff in einer einmaliger Dosis von 5 bis 50 mg. Das erfindungsgemässe Arzneimittel in Form von Kapseln enthält vorzugsweise einen Wirkstoff in einer Menge von 5 bis 50 mg in einer Kapsel und als pharmazeutische Trägersubstanz Olivenöl.

## Beste Ausführungsvariante der Erfindung

Antiulzeröse Wirkung des erfindungsgemässen Hexapeptids wurde in Versuchen an Tieren untersucht.

Die Untersuchung der antiulzerösen Wirkung erfolgte in Versuchen an Rattenmännchen der Linie Wistar im Vergleich zu den bekannten Präparaten.

Die Entwicklung der ulzerösen Schädigungen des Zwölffingerdarms bei Ratten erfolgte durch subkutane Injektion des Zysteaminhydrochlorids in einer Dosis von 350 mg/kg, das in 0,5 ml physiologischer Lösung aufgelöst ist. Gleich nach der Einführung des Zysteaminhydrochlorids und in 6 Stunden wurde den Ratten mit Hilfe einer Magensonde das erfindungsgemässe Hexapeptid enteral in einer Dosis von 10 und von 100 µg/kg, oder bekannten Hexapeptid (US, A, 4565805) in einer Dosis von 100 µg/kg beziehungsweise Zimetidin in einer Dosis von 10 µg/kg, die in 0,5 ml 3%iger Milch aufgelöst sind, die fünffach mit physiologischer Lösung verdünnt ist, eingeführt. Der Kontrollgruppe von Tieren führte man gleich nach der Verabreichung des Zysreaminhydrochlorids und in 6 Stunden enteral 0,5 ml 3%ige Milch ein, die fünffach mit physiologischer Lösung verdünnt ist. Das Töten der Tiere erfolgte durch Dekapitation in 24 Stunden nach der Einführung des Zysteaminhydrochlorids und man bewertete den Zustand der Schleimhaut des Zwölffingerdarmes.

Für jede Gruppe von Ratten errechnete man den durchschnittlichen Befallsgrad, der in Punkten ausgedrückt wird (0 - Ausbleiben von erosion-ulzerösen Schädigungen, 1 - Erosionen, 2 - vereinzeltes Magengeschwür, 3 - multiplexe Magengeschwüre, 4 - perforierende Magengeschwüre). In jeder Gruppe ermittelte man ausserdem die Frequenz der Schädigungen: das Verhältnis der Anzahl der Tiere mit Magengeschwüren zu der Gesamtanzahl der zum Ende des Versuches am Leben gebliebenen Tiere: Als Gesamtkennziffer der Magengeschwürbildung ermittelte man den bezogenen Magengeschwürfaktor (die Schwäre des Befalls + verdoppelte Befallsfrequenz).

Statische Bearbeitung der erzielten Ergebnisse erfolgte unter Zuhilfenahme des t-Studentkriteriums

(für die Schwere des Befalls) und des $x^2$-Pirsonkriteriums (für Befallsfrequenz). Als zuverlässige Kennziffern betrachtete man die Differenzen bei Werten von 95% (bei $P < 0,05$). Die erzielten Ergebnisse sind in der Tabelle 1 angeführt.

Tabelle 1

Wirkung des erfindungsgemaßen Hexapeptids, des Zymetidins und des bekannten antiulzerösen Hexapeptids auf die Entstehung von Zysteamin-Zwölffingerdarmgeschwüren bei enteraler Verabreichung

| lfd. Nr. | Kennziffern | Kontrollgruppe | Erfindungsgemäßes Hexapeptid (10 μ g/kg) |
|---|---|---|---|
| 1 | 2 | 3 | 4 |
| 1. | Schwere des Befalls (in Punkten) | 2,17 ± 0,15 | 1,60 ± 0,27 |
| 2. | Befallsfrequenz | 0,86 | 0,80 |
| 3. | Ulkus-Faktor | 3,89 | 3,20 |
| 4. | Senkung des Ulkus--Faktor (in %) | - | 18 |
| 5. | Anzahl der Tiere | 42 | 10 |

Fortsetzung der Tabelle 1

| lfd. Nr. | Erfindungsgemäßes Hexapeptid (100 μ g/kg) | Zymetidin (10 μ mg/kg) | Bekanntes antiulzeröses Hexapeptid (100 μg/kg) |
|---|---|---|---|
| 1 | 5 | 6 | 7 |
| 1. | 0,83 ± 0,17* | 1,00 ± 0,26* | 1,62 ± 0,19 |
| 2. | 0,31* | 0,33* | 0,65 |
| 3. | 1,45 | 1,66 | 2,92 |
| 4. | 63 | 57 | 26 |
| 5. | 36 | 12 | 26 |

* Differenz zur Kontrollgruppe bei Werten von 95% (bei $P < 0,05$).

Wie aus der Tabelle 1 zu ersehen ist, verhinderte das erfindungsgemässe Hexapeptid bei der peroralen Verabreichung in Abhängigkeit von der Dosis die Geschwürbildung im Zwölffingerdarm von Ratten und setzte den Ulkusfaktor bei einer Dosis von 100 μg/kg um 63% herab. Die antiulzeröse Wirkung des erfindungsgemässen Hexapeptids war höher als die antiulzeröse Wirkung des Zymetidins (in einer Dosis von 10 mg/kg), das den Ulkusfaktor um 57% verringerte. Bekanntes antiulzeröses Hexapeptid (US, A, 4565805) wies bei peroraler Verabreichung eine schwache antiulzeröse Wirkung auf, und setzte den Ulkusfaktor lediglich um 26% herab.

Das erfindungsgemässe Hexapeptid wurde auch an einem Versuchsmodell von Indometacinum-Geschwüren des Dünndarmes untersucht, an dem man die antiulzeröse Wirkung des Hexapeptids mit der antiulzerösen Wirkung des Sulfasalazins verglich. Indometacinum führte man den Ratten peroral in einer Dosis von 20 mg/kg unter Zuhilfenahme einer Magensonde in Form einer Suspension in 0,5 ml physiologischer Lösung ein. Das erfindungsgemässe Hexapeptid in einer Dosis von 100 μg/kg beziehungsweise Sulfasalazin in einer Dosis von 200 mg/kg führte man gleich und in 6 Stunden nach der Verabreichung des Indometacinums in 0,5 ml der fünffach mit der physiologischen Lösung verdünnten 3%igen Milch ein. Der Kontrollgruppe der Tiere führte man gleich und in 6 Stunden nach der Verabreichung des Indometacinums 0,5 ml der 3%igen Milch peroral ein, die fünffach mit physiologischer Lösung verdünnt ist. Das Töten der Tiere durch die Dekapitation erfolgt in 24 Stunden nach der Verabreichung des Indometacinums, und man errechnete die Anzahl von Geschwürbeschädigungen der Schleimhaut des 30 cm langen distalen Abschnitts des Dünndarmes, in dem die Entstehung von Geschwüren maximal war. Für jede Gruppe von Tieren ermittelte man

die durchschnittliche Anzahl von Geschwüren im Darm.

Die statische Bearbeitung der erzielten Ergebnisse erfolgt mit Hilfe des t-Studentkriteriums. Als zuverlässig galten die Differenzen bei Werten von 95% (bei $P < 0,05$). Die erzielten Ergebnisse sind in der Tabelle 2 angeführt.

Tabelle 2

Wirkung des erfindungsgemässen Hexapeptids und des Sulfasalazins auf die Entwicklung von Indometacinum-Geschwüren in Dünndarm von Ratten

| Gruppen von Tieren | Anzahl der Tiere | Durchschnittliche Anzahl der Geschwüre |
|---|---|---|
| Kontrolle | 20 | 48,78±6,02 |
| Hexapeptid | 20 | 23,40±5,36* |
| Kontrolle | 12 | 50,78+5,81 |
| Sulfasalazin | 12 | 27,50±5,28* |

* Unterschied zur Kontrollgruppe ist zuverlässig bei Werten von 95% ($P < 0,05$).

Wie aus der Tabelle 2 zu ersehen ist, verringert das erfindungsgemässe Hexapeptid in einer Dosis von 100 µg/kg die durchschnittliche Anzahl der Geschwüre im distalen Abschnitt des Dünndarmes von Ratten um 52%. Die antiulzeröse Wirkung des erfindungsgemässen Hexapeptids steht nicht der antiulzerösen Wirkung des Sulfasalazins nach, das in einer Dosis von 200 mg/kg verabreicht wurde und das die Anzahl der Geschwüre im Darm um 46% herabsetzt.

In allen Versuchen mit Verwendung des erfindungsgemässen Hexapeptids wurden keine Veränderungen seitens des zellulären Zusammensetzung des Blutes, der wichtigsten Hämodynamischen Kennziffern nachgewiesen. Bei der Untersuchung der akuten Toxizität des erfindungsgemässen Hexapeptids betrug $LD_{50}$ 250 mg/kg.

Hierdurch stand das erfindungsgemässe Hexapeptid in den eingesetzten Versuchmodellen einer akuten erosion-ulzerösen Beschädigung des Magen-Darm-Traktes nicht dem Żymetidin (in bezug auf den Zwölffingerdarm) und dem Sulfasalazin (in bezug auf den Dünndarm) nach. Da das Zymetidin in einer Dosis von 10 mg/kg und das Sulfasalazin in einer Dosis von 200 mg/kg verabreicht wurden, ging daraus hervor, dass die antiulzeröse Wirkung des erfindungsgemässen Hexapeptids in den verwendeten Versuchsmodellen höher als die Wirkung des Zymetidins und des Sulfasalazins auf das 100fache bzw. auf das 2000fache ist.

Das erfindungsgemässe Hexapeptid, das in seiner Wirkung das Zymetidin und das Sulfasalazin übertrifft, findet Anwendung als Wirkstoff in einem Arzneimittel für die Behandlung der erosion-ulzerösen Beschädigungen des Magen-Darm-Traktes (Magengeschwüre, Zwölffingerdarmgeschwüre und Colitis ulzerosa). Als vorteilig bei der Verwendung des erfindungsgemässen Präparats im Vergleich zum Zymetidin und Sulfasalazin erscheint eine höhere Wirkung und die Möglichkeit der Anwendung bei der gleichzeitigen Erosionsbeschädigung des Zwölffingerdarms und der distalen Abschnitte des Dünndarms, und als vorteilig im Vergleich zu dem bekannten antiulzerösen Hexapeptid (US, A, 4565805) erscheint die Möglichkeit seiner peroralen Verabreichung.

Bei der Behandlung der Patienten mit erosion-ulzerösen Beschädigungen der Schleimhaut des Magen-Darm-

Traktes kann das erfindungsgemässe Arzneimittel in verschiedenen Arzneiformen für die perorale Verabreichung in einer einmaligen Dosis von 5 bis 50 mg angewendet werden.

Das erfindungsgemässe Arzneimittel kann in beliebiger Arzneiform, die für perorale Verabreichung geeignet ist, beispielsweise, in Form von Pulvern beziehungsweise von Tabletten für die perorale Verabreichung in Milch, in Pflanzenfetten oder Lipiden in disperser Form sowie in Form von Kapseln, die beliebige pharmazeutisch geeignete Fettträgersubstanz enthalten, verwendet werden. Das erfindungsgemässe Präparat kann in Form eines intranasalen Aerosols beziehungsweise von Rektalzöpfchen verwendet werden.

Erfindungsgemässes Hexapeptid folgender Formel:

Tyr - D - Ala - Gly - Phe - Leu - Glu

synthetisiert man aus zwei Fragmenten, die durch ein konsequentes Angliedern der Aminosäurekette mit je einer Aminosäure: di-tert.-Butylester der Karbobenzoxi-phenylalanyl-leuzylglutaminsäure ausgehend von dem Di-tert.-butylester der Karbobenzoxy-glutaminsäure; N-tert.-Butyloxi-karbonyl-O-benzyltyrosil-D-alanylglyzin ausgehend vom Natriumsalz des Glyzins, und aktivierten Estern der geschützten Aminosäuren. Die Fragmente wurden nach der Beseitigung der Karbobenzoxy-Gruppe nach der Methode der aktivierten Ester kondensiert. Die Schutzgruppen wurden mit Hilfe der katalytischen Hydrogenolyse und der azidolytischen Spaltung entfernt.

Zur besseren Erläuterung der vorliegenden Erfindung wird nachstehendes Beispiel für die Herstellung des erfindungsgemässen Hexapeptids angeführt.

Beispiel 1

6,83 g (17,41 μMol) Di-tert.-butylester der Karbobenzoxy-glutaminsäure löst man in 35 ml Methanol

und hydriert in Gegenwart eines Palladiumkatalysators innerhalb von 4 Stunden. Der Katalysator wird abgefiltert, an einem Filter mit Methanol gewaschen, das Filtrat wird eingedampft und in 25 ml Dimethylformamid aufgelöst. Der Lösung fügt man 4,50 g (17,41 µMol) p-Nitrophenyläther des Karbobenzoxyleuzins hinzu. Das Reaktionsgemisch vermischt man bei Raumtemperatur innerhalb von 24 Stunden. Das Lösungsmittel dampft man ein, den Rückstand löst man in IO ml eines Gemisches Äthylazetat:Hexan (2:1) auf und trägt man auf eine Chromatografiesäule auf. Die Chromatografie führt man unter isokratischen Bedingungen (Eluent - Äthylazetat:Hexan 2:1) durch. Fraktionen werden eingedampft und in einem Vakuumexikator getrocknet. Man erhält 6,02 g (68%) Di-tert.-butylesters der Karbobenzoxy-leuzyl-glutaminsäure als farbloses Öl, $[\alpha]_D^{20}$ = -24,2° (c 1; Dimethylformamid), $R_f$ : 0,22 (Äthylazetat-n-hexan 1:2); 0,6I (Toluol:Azeton:Methanol:Essigsäure 14:1:4:1), 0,50 (Toluol:n-Heptan:Methyläthylketon 5:3:1).

1,56 g (3,09 µMol) Di-tert.-butylester der Karbobenzoxy-leuzyl-glutaminsäure löst man in 10 ml Methanol und hydriert man in Gegenwart eines Palladiumkatalysators innerhalb von 3 Stunden. Der Katalysator wird abgefiltert, an einem Filter mit Methanol gewaschen, und das Filtrat wird eingedampft. Der Rückstand wird in 5 ml Dimethylformamid aufgelöst, man setzt 1,30 g (3,09 µMol) p-Nitrophenyläther des Karbobenzoxyphenylalanin zu . Das Reaktionsgemisch wird bei Raumtemperatur innerhalb von 24 Stunden vermischt. Das Lösungsmittel dampft man ein, den Rückstand löst man in 30 ml Äthylazetat auf. Äthylazetatlösung wäscht man mit 2%iger Schwefelsäure (20 ml x 2) und mit Wasser (20 ml x 2), dampft man ein, setzt man 25 ml Isopropylalkohol hinzu und dampft man ein. Den

Rückstand löst man in Di-isopropyläther auf, die ausgefallenen Kristalle filtert man ab, wäscht man an einem Filter mit Di-isopropyläther und trocknet man in einem Vakuumexikator. Man erhält 1,55 g (85) Di-tert.-butylester der Karbobenzoxy-phenylalanyl-leuzyl-glutaminsäure vom Schwelzpunkt 120°C, $[\alpha]_D^{20}$ = -25,6° (c 1, Dimethylformamid), $R_f$ : 0,23 (Äthylazetat:n-Hexan 1:2), 0,56 (Toluol-Azeton-Methanol-Essigsäure 14:1:4:1), 0,29 (Toluol:n-Heptan-Methyläthylketon 5:3:1).

0,50 g (7,68 µMol) Glyzin löst man in 7,68 ml 1 n-Ätznatronlösung auf und setzt man eine Lösung von 2,74 g (7,78 µMol) p-Nitrophenyläther des Karbobenzoxy-D-alanins in 20 ml Dimethylformamid zu . Das Reaktionsgemisch vermischt man bei Raumtemperatur innerhalb von 24 Stunden. Die Lösung dampft man ein, den Rückstand löst man in Wasser auf, extrahiert mit Äther (50 ml x 3), die wässerige Schicht säuert man mit 2 n-Schwefelsäure bis zur Erzielung eines pH-Wertes von 2 an und extrahiert mit Äthylazetat (50 ml x 3). Die Äthylazetatlösung wäscht man mit Wasser (30 ml x 2), setzt man 50 ml Isopropylalkohol hinzu und dampft man ein. Den Rückstand giesst man mit Äther (100 ml) über, die entstandenen Kristalle filtert man ab, wäscht an einem Filter mit Äther und trocknet in einem Vakuumexikkator.

Man erhält 1,40 g (65%) Karbobenzoxy-D-alanyl-glyzin mit einem Schmelzpunkt von 126,5 bis 127°C, $[\alpha]_D^{20}$ = +20,7 (c 1, MeOH), $R_f$ : 0,67 (Chloroform: Methanol - 32%ige Essigsäure 60:45:20); 0,7I (Äthylazetat-Pyridin:Essigsäure:Wasser 45:20:6:11); 0,35 (Methylenchlorid:Methanol:50%ige Essigsäure 85:15:2).

1,66 g (5,90 µMol) Karbobenzoxy-D-alanyl-glyzin löst man in 20 ml Methanol auf und hydriert in Gegenwart eines Palladiumkatalysators innerhalb von 3 Stunden. Der Katalysator wird abgefiltert, an einem Filter mit Methanol gewaschen, das Filtrat wird eingedampft, der Rückstand in 5,9 ml 1 n-Ätznatronlösung aufgelöst, der Lösung setzt man 2,91 g (5,90 $\mu$Mol) p-Nitrophenyläther des N-tert.-Butyloxykarbonyl-O-benzyl-tyrosins in 25 ml Dimethylformamid zu. Das Reaktionsgemisch vermischt man bei Raumtemperatur innerhalb von 24 Stunden. Die Lösung dampft man ein, den Rückstand löst man im Wasser auf, extrahiert mit Äther (50 ml x 2), die wässerige Lösung säuert man mit 2 n-Schwefelsäure bis zum Erzielen eines pH-Wertes von 2 an, extrahiert man mit Äthylazetat (70 ml x 3). Extrakt wird mit Wasser (50 ml x 2) gewaschen, man setzt 50 ml Isopropylalkohol hinzu und dampft man ein. Den Rückstand giesst man mit Äther (150 ml) über, die entstandenen Kristalle filtert man ab, wäscht man mit Äther an einem Filter und trocknet in einem Vakuumexikkator. Man erhält 2,62 g (89%) N-tert. -Butyloxykarbonyl-O-benzyl-tyrosil-D-alanyl-glyzin mit einem Schmelzpunkt von 100 bis 100,5°C, $[\alpha]_D^{20} = -9,3^\circ$ (c 1, Dimethylformamid), $R_f$ : 0,74 (Äthylazetat:Pyridin-Essigsäure:Wasser 45:20:6:11); 0,57 (Methylenchlorid:Methanol 50%ige Essigsäure 85:15:2); 0,36 (Chloroform:Methanol:Essigsäure 9:1:0,5).

3,50 g (7,03 $\mu$Mol) N-tert.-Butyloxykarbonyl-O-benzyl-tyrosyl-D-alanyl-glyzin und 1,02 g (7,33 $\mu$Mol) p-Nitrophenol löst man in 25 ml Dimethylformamid auf, die Lösung kühlt man auf -25°C ab, setzt man 1,54 g (7,33 $\mu$Mol) Dizyklohexylkarbodiimid in 10 ml Dimethylformamid zu. Das Reaktionsgemisch vermischt man bei einer Temperatur von -25°C innerhalb von 45 Minuten, dann bringt man die Temperatur auf 0°C und hält man bei dieser Temperatur innerhalb von 24 Stunden. Die ausgefallenen Kristalle des Dizyklohexylharnstoffes filtert man ab, wäscht man an einem Filter

mit Dimethylformamid, dampft das Filtrat ein, den Rückstand kristallisiert man aus dem Isopropylalkohol um.

Man erhält 3,63 g (83%) p-Nitrophenyläther des N-tert.-Butyloxy-karbonyl-O-benzyltyrosyl-D-alanyl-glizins mit einem Schmelzpunkt von 154°C, $[\alpha]_D^{20} = -0,4^\circ$ (c 1, Dimethylformamid), $R_f$ : 0,87 (Methylenchlorid:Methanol:50%ige Essigsäure 85:15:2), 0,69 (Chloroform:Methanol-Essigsäure 32:3:1), 0,41 (n-Butanol-Ameisensäure:Wasser 15:3:1).

209 mg (0,32 µMol) Di-tert. - butylester der Karbobenzoxy-phenylalanyl-leuzyl-glutaminsäure löst man in 15 ml Methanol auf und hydriert in Gegenwart eines Palladiumkatalysators innerhalb von 3 Stunden. Den Katalysator filtert man ab, wäscht an einem Filter mit Methanol, das Filtrat dampft man ein.

Den Rückstand löst man in 10 ml Dimethylformamid und setzt man 200 mg p-Nitrophenyläther des N-tert.-Butyloxykarbonyl-O-benzyl-tyrozyl-D-alanyl-glyzins zu. Das Reaktionsgemisch vermischt man bei Raumtemperatur innerhalb von 24 Stunden. Das Dimethylformamid dampft man ein, den Rückstand fällt man zweimal aus Methanol (5 ml) mit Äther (300 ml) um. Man erhält 266 mg (84%) Di-tert.-butylesters der N-tert.-Butyloxykarbonyl-O-benzyl-tyrosyl-D-alanyl-glyzyl-phenylalanyl-leuzyl-glutaminsäure mit einem Schmelzpunkt von 200 bis 201°C, $[\alpha]_D^{20} = -19,2^\circ$ (c 1, Dimethylformamid), $R_f$ : 0,88 (Chloroform:Methanol:32%ige Essigsäure 60:45:20); 0,89 (Äthylazetat:Pyridin:Essigsäure:Wasser 45:20:6:11); 0,58 (Chloroform:Methanol:Essigsäure 9:1:0,5).

240 mg (0,24 µMol) Di-tert.-butylester der N-tert.-Butyloxykarbonyl-O-benzyl-tyrozyl-D-alanyl-glyzyl-phenylalanyl-leuzyl-glutaminsäure löst man in 15 ml Essigsäure auf und hidriert man in Gegenwart eines Palladiumkatalysators innerhalb von 3 Stunden. Den Katalysator filtert man ab, wäscht man an einem

Filter mit Essigsäure und das Filtrat dampft man ein. Den Rückstand löst man in 15 ml Trifluoressigsäure auf, hält innerhalb von 30 Minuten bei Raumtemperatur. Die lösung dampft man ein, den Rückstand giesst man mit 50 ml Äther über, den ausgefallenen Niederschlag filtert man ab, wäscht man an einem Filter mit Äther, löst man in 50 ml 10%iger Essigsäure auf, behandelt mit Ionenaustauscherharz "Dowex-1" in $AcO^-$-Form. Das Harz filtert man ab, wäscht man an einem Filter mit 10%iger Essigsäure, das Filtrat dampft man ein. Das hergestellte Produkt reinigt man im Gegenstrom-Verteilungsverfahren in einer Planeten-Zentrifuge im Sytem n-Butanol:Essigsäure:Wasser 4:1:5; stationäre Phase ist organische Phase. Die Fraktionen, die das Endprodukt enthalten, sammelt man, dampft man ein, den Rückstand fällt man aus Methanol (3 ml) mit Äther (250 ml) um, den entstandenen Niederschlag filtert man ab, wäscht man an einem Filter mit Äther und trocknet man in einem Vakuumexikkator.

Man erhält 92 mg (55%) Tyrosyl-D-alanyl-glyzyl-phenyl-alanyl-leuzyl-glutaminsäure, $[\alpha]_D^{20} = +29,8°$ (c 1, 10% AcOH), $R_f$ : 0,74 (Chloroform:Methanol:32%ige Essigsäure 60:45:20); 0,42 (Äthylazetat-Pyridin-Essigsäure:Wasser 45:20:6:11); 0,48 (n-Butanol-Essigsäure:Wasser 3:1:1). Die Angaben der Animosäure-Analyse : Alanin 1,02 (1), Glyzin 1,00 (1), Glutaminsäure 1,04 (1), Leuzin 1,02 (1), Tyrosyl 0,99 (1), Phenyl-alanin 1,03 (1). Die Angaben einer hocheffektiven Flüssigchromatografie : Peptid wurde von einem Berg bei 38,24% des Gradienten in der 12. und in der 16. Minute eluiert, Säule 250 x 4,6 mm. Ultrasphera ODS 5 m, bewegliche Phase A: 0,05 Mol $KH_2PO_4$, B:$CH_3CN$ grad 20 → 50% B in 20 Minuten, Druck 1500psi, Geschwindigkeit 1 ml/min, Detektion bei 214 nm.

Industrielle Anwendbarkeit

Das erfindungsgemässe Hexapeptid bezitzt eine antiulzeröse Wirkung und kann in der Medizin als Wirkstoff eines Arzneimittels für die Behandlung von erosion-ulzerösen Schädigungen des Magen-Darm-Traktes Anwendung finden.

PATENTANSPRÜCHE:

1. Hexapeptid folgender Formel:

Tyr - D - Ala - Gly - Phe - Leu - Glu

2. Arzneimittel für die Behandlung von erosion-ulzerösen Schädigungen des Verdauungstraktes, das einen Wirkstoff und eine pharmazeutische Trägersubstanz vorsieht, dadurch gekennzeichnet, dass es als Wirkstoff Hexapeptid nach Anspruch 1 enthält.

3. Arzneimittel nach Anspruch 2, dadurch gekennzeichnet, dass es den Wirkstoff in einer einmaligen Dosis von 5 bis 50 mg enthält.

4. Arzneimittel nach Anspruch 2 in Form von Kapseln, dadurch gekennzeichnet, dass es den Wirkstoff in einer Menge von 5 bis 50 mg in einer Kapsel enthält.

5. Arzneimittel nach Anspruch 4, dadurch gekennzeichnet, dass es als pharmazeutische Trägersubstanz Olivenöl enthält.

08906225.1

00-7C

0341311

# INTERNATIONAL SEARCH REPORT

International Application No PCT/SU 88/00115

**I. CLASSIFICATION OF SUBJECT MATTER** (if several classification symbols apply, indicate all) [6]

According to International Patent Classification (IPC) or to both National Classification and IPC

$IPC^4$ C07K 7/06,A61K 9/48,35/78,37/02

**II. FIELDS SEARCHED**

Minimum Documentation Searched [7]

| Classification System | Classification Symbols |
|---|---|
| $IPC^4$ | C07K 7/06, A61K 9/48,35/78,37/02 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | EP, A1, 0189485 (VSESOJUZNY KARDIOLOGICHESKY NAUCHNY TSENTR AKADEMII MEDITSINSKIKH NAUK SSSR) 6 August 1986 (06.08.86) see the abstract & SU, A1, 1348343 | 1,2 |
| A | WO, A1, 84/02470 (Vsesojuny kardiologichesky tsentr Akademii meditsinskikh nauk SSSR) 5 July 1984 (05.07.84) see the claims & FR, A1, 2541115 CH, A5, 661869 | 1-5 |
| A | US, A, 4457917 (Max-Planck-Gesellschaft zur Foerderung der Wissenschaften e.v.), 3 July 1984 (03.07.84) see the abstract | 1-5 |
| A | EP, A2, 0187956 (Boehringer Ingelheim International G.m.b.H.) 23 July 1986 (23.07.86) see the claims | 2-5 |

./.

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 2 September 1988 (02.09.88) | 5 October 1988 (05.10.88) |
| **International Searching Authority** | **Signature of Authorized Officer** |
| ISA/SU | |

International Application No PCT/SU 88/00115

FURTHER INFORMATION CONTINUED FROM THE SECOND SHEET

| | | |
|---|---|---|
| A | EP, A1, 0188629 (VSESOJUZNY KARDIOLOGICHESKY NAUCHNY TSENTR AKADEMII MEDITSINSKIKH NAUK SSSR) 30 July 1986 (30.07.86) see the abstract | 1,2 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers.........., because they relate to subject matter not required to be searched by this Authority, namely:

2.☐ Claim numbers.........., because they relate to parts of the international application that do not comply with the prescribed requirements to such an extent that no meaningful international search can be carried out, specifically:

3.☐ Claim numbers.........., because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.